(19)

**Europäisches Patentamt**
**European Patent Office**
**Office européen des brevets**

(11)  **EP 4 425 446 A1**

(12)  # EUROPEAN PATENT APPLICATION

(43) Date of publication:
**04.09.2024 Bulletin 2024/36**

(21) Application number: **24160837.1**

(22) Date of filing: **01.03.2024**

(51) International Patent Classification (IPC):
**G06V 10/82** *(2022.01)*    **G06V 20/69** *(2022.01)*

(52) Cooperative Patent Classification (CPC):
**G06V 10/82; G06V 20/693; G06V 20/698**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.03.2023 US 202363487715 P**
**02.05.2023 US 202363499645 P**

(71) Applicants:
• **FEI Company**
**Hillsboro, OR 97124-5793 (US)**
• **Brammer Bio, LLC.**
**Alachua, FL 32615 (US)**

(72) Inventors:
• **PHELIPPEAU, Harold**
**33 800 Bordeaux (FR)**
• **BRESSERS, Martinus**
**33 800 Bordeaux (FR)**
• **KAIRAWICZ, Laure Helene**
**Alachua, 32615 (US)**
• **MILIAN, Steven**
**Alachua, 32615 (US)**

(74) Representative: **Boult Wade Tennant LLP**
**Salisbury Square House**
**8 Salisbury Square**
**London EC4Y 8AP (GB)**

(54)  **SUPERVISED MACHINE LEARNING BASED CLASSIFICATION OF ADENO ASSOCIATED VIRUSES IN CRYOGENIC ELECTRON MICROSCOPY (CRYO-EM)**

(57)    Disclosed herein are scientific instrument support systems, as well as related methods, computing devices, and computer-readable media. For example, in some embodiments, a scientific instrument support apparatus may include: first logic to receive, from a cryogenic (Cryo) electron microscopy, cryo-microscopy (Cryo-EM) data regarding a biological specimen having a plurality of capsids; second logic to determine a classification for each of the capsids by processing the Cryo-EM data through an AI model trained under various acquisition conditions; and third logic to display the classifications of the capsids in the biological specimen.

**EP 4 425 446 A1**

**Description**

Cross-Reference to Related Applications

This application claims the priority of US Provisional Application 63/487,715, filed on March 1, 2023 and US Provisional Application 63/499,645, filed on May 2, 2023, the disclosure of which is incorporated herein by reference in its entirety.

Background

Microscopy is the technical field of using microscopes to better view objects that are difficult to see with the naked eye. One branch of microscopy, known as charged particle (e.g., electron and/or ion) microscopy, involves using a beam of accelerated charged particles as a source of illumination. Types of charged particle microscopy include, for example, transmission electron microscopy (TEM), scanning electron microscopy (SEM), scanning transmission electron microscopy, and ion beam microscopy.

Brief Description of the Drawings

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.

FIG. 1 is a block diagram of an example scientific instrument support module for determining a classification for each of a plurality of capsids in a biological specimen, in accordance with various embodiments.
FIG. 2A is a flow diagram of an example method to determine a classification for each of a plurality of capsids in a biological specimen, in accordance with various embodiments.
FIG. 2B is a flow diagram of an example method of generating and selecting an artificial intelligence (AI) model for determining a classification for each of a plurality of capsids in a biological specimen, in accordance with various embodiments.
FIG. 3A depicts micrographs containing empty, partial, and full capsids.
FIG. 3B depicts an example of an empty capsid, a partially filed capsid, and a full capsid.
FIG. 4A depicts a principal component analysis (PCA) clustering.
FIG. 4B depicts silhouette scores corresponding to the PCA clustering depicted in FIG. 4A.
FIG. 5A depicts a chart showing a radial averaged density profile of averaged classes.
FIG. 5B depicts a chart showing a distribution of average inner density per class.

FIG. 6 is an example of a graphical user interface that may be used in the performance of some or all of the support methods disclosed herein, in accordance with various embodiments.
FIG. 7 is a block diagram of an example computing device that may perform some or all of the scientific instrument support methods disclosed herein, in accordance with various embodiments.
FIG. 8 is a block diagram of an example scientific instrument support system in which some or all of the scientific instrument support methods disclosed herein may be performed, in accordance with various embodiments.

Detailed Description

Disclosed herein are scientific instrument support systems, as well as related methods, computing devices, and computer-readable media. For example, in some embodiments, a scientific instrument support apparatus may include: first logic to receive, from a cryogenic (Cryo) electron microscope, cryogenic electron microscopy (Cryo-EM) data regarding a biological specimen having a plurality of capsids; second logic to determine a classification for each of the capsids by processing the Cryo-EM data through an artificial intelligence (AI) model trained with annotated data; and third logic to display the classifications of the capsids in the biological specimen.

Gene therapy holds great promise for a wide range of diseases, including cancers, cardiovascular disease and thousands of rare hereditary diseases caused by genetic mutations. Viruses have a natural ability to deliver genetic material into cells, and, once viruses have been modified to eliminate their ability to cause infectious diseases, these modified viruses can be used as vectors to transport therapeutic genes into human cells. In recent years, interest in gene therapy has accelerated with new gene therapies approved by the United States Food and Drug Administration in 2020 as well as many new therapies under development, resulting in a high demand for viral vectors. This growing interest in gene therapy has led to the need for more cost-effective and scalable viral vector manufacturing platforms to provide these therapies. The adeno associated virus (AAV) is routinely used in many manufacturing platforms based on, for example, its safety profile, scalability, effectiveness, and low cost. For example, along with lentiviral vectors, the AAV is used in roughly 90% of gene therapies. However, AAV-based gene therapies are more complex than many traditional biotherapies.

Generally, AAV is formed by a closed viral capsid encapsulating a single-stranded deoxyribonucleic acid (DNA). Three types of capsids are produced during the production of AAV vectors: empty, partially filled, and filled. Empty capsids are considered impurities. Lacking access to data, partially filled may be considered impurities. In addition to the complete capsid containing the desired genetic material, the final product may contain many types of impurities related to the product. The presence of impurities can affect the efficacy and safety of AAV vector products as they increase the risk of immune response to the product. In addition, the presence of empty or partially filled particles can obstruct the transduction of full AAV capsids by competing for vector binding sites when administered to patients. Therefore, the amount of these impurities and of the desired filled AAV capsids or proper dosing of patient needs to be determined. In addition to full capsids, the final product may contain some types of process and product impurities. Accordingly, one fundamental challenge in the manufacture of virus-based products (gene therapy, vaccines based on viral-like particles) is to achieve sufficient purity while maintaining the integrity of the virus.

Cryo-EM is a method by which biological specimens are vitrified by rapid freezing, thus integrating molecules into an amorphous ice. The samples thus prepared preserve their native structure. There are no markers or other chemicals. If a capsid contains DNA, there will be an internal density of dark pixels, and, if the capsid does not contain DNA, the internal density will be close to the background of the image. Cryo-EM can provide unique information and provide access to multiple attributes, such as, for example, spatial distribution, capsid integrity, or a combination thereof.

Artificial intelligence has been proven to provide state of the art results in the domain of computer vision for object detection and classification. However, training an AI model requires ground truth annotated data, and this step becomes a bottleneck when the classification is difficult or impossible by the naked eye, such as, for example, when looking at Cryo-EM images of products containing capsids. For example, annotation (differentiation) of partial/full capsids is extremely difficult with the naked eye. In some embodiments, the systems and methods described herein solve this problem using supervised machine learning models. As discussed in further detail below, in some embodiments, the methods and systems train a model with data augmentation using previously annotated data. One described system provides supervised annotation of micrographs containing empty/partial/full capsids with machine learning approaches. In some embodiments, a generally trained AI model for classifying empty/partial/full capsids classification is trained under various acquisition conditions. Acquisition conditions may include settings (e.g., defocus, magnification, electron dose, exposure time, and the like), types of acquisition systems (e.g., cryogenic electron transmission microscopes), models of microscopes, or a combination thereof. In some embodiments, the AI model is trained, from a large amount of annotated data, to be generalized in the sense that it can perform well in all situations (e.g., contrast change, noise, magnification, and the like). In some embodiments, the training of the model is reinforced by using data augmentation such as artificially modified annotated data that includes, for example, contrast change, flipping, rotations, and the like.
In some embodiments, the described systems and methods determine the purity of AAV based products in the pharma industry during the product development phase or for quality control during the production phase. In some embodiments, the systems and methods are employed to annotate micrographs of reference stocks of empty/partial/full capsids even when partial and full capsids cannot be distinguished by the naked eye. In some embodiments, the model is trained using data augmentation and can thus be generalized to predict the percentage of empty/partial/full capsids on various microscopes and various acquisition conditions. Accordingly, the scientific instrument support embodiments disclosed herein may achieve improved performance relative to conventional approaches. For example, embodiments described herein use machine learning to efficiently annotate micrographs of reference stocks of empty, partial, and full capsids that cannot be otherwise distinguished by the naked eye, which allows for the efficient creation of ground truth for training an AI model. The annotated data can be associated with different acquisition conditions, which allows the AI model to be generalized to predict the percentage of empty, partial, and full capsids on various microscopes and under various acquisition conditions, which avoids the need for creating separate AI models (which would result in inefficient use of computing resources). The embodiments disclosed herein thus provide improvements to scientific instrument technology (e.g., improvements in the computer technology supporting such scientific instruments, among other improvements) through the use of annotation of images for use in training an AI model, which may be used to detect impurities in gene therapy products. Accordingly, embodiments disclosed herein improve gene therapy products and the manufacturing process thereof and may achieve improved speed, accuracy, throughput, cost efficiency, and reliability relative to conventional approaches. Such technical advantages are not achievable by routine and conventional approaches, and all users of systems including such embodiments may benefit from these advantages (e.g., by assisting the user in the performance of a technical task, such gene therapy product manufacture, by means of an automated process using AI models). The technical features of the embodiments disclosed herein are thus decidedly unconventional in the field of gene therapy techniques, as are the combinations of the features of the embodiments disclosed herein. As discussed further herein, various aspects of the embodiments disclosed herein may improve the functionality of a computer itself; for example, as described herein, computing devices employing machine learning to classify capsids, and can improve their accuracy for future operations by receiving feedback on their past results. The computational and user interface features disclosed herein do not only involve the collection and comparison of information but apply new analytical and technical techniques to change the operation of scientific instruments used in genetic product manufacture. The present disclosure thus introduces functionality that neither a conventional computing device, nor a human, could perform.

Accordingly, the embodiments of the present disclosure may serve any of a number of technical purposes, such as controlling a specific technical system or process (e.g., an AAV quality control system or process) and providing image enhancement or analysis for use in, among other things, gene therapy products. In particular, the present disclosure provides technical solutions to technical problems, including but not limited to, automated annotation of images not available through manual processing and use of such annotated images to train an AI model for improved classification of capsids. The embodiments disclosed herein thus provide improvements to gene therapy product manufacture and technology associated with scientific instruments (e.g., improvements in the computer technology supporting gene therapy product manufacture, among other improvements).

In the following detailed description, reference is made to the accompanying drawings that form a part hereof wherein like numerals designate like parts throughout, and in which is shown, by way of illustration, embodiments that may be practiced. It is to be understood that other embodiments may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described embodiment. Various additional operations may be performed, and/or described operations may be omitted in additional embodiments.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices.

The description uses the phrases "an embodiment," "various embodiments," and "some embodiments," each of which may refer to one or more of the same or different embodiments. Furthermore, the terms "comprising," "including," "having," and the like, as used with respect to embodiments of the present disclosure, are synonymous. When used to describe a range of dimensions, the phrase "between X and Y" represents a range that includes X and Y. As used herein, an "apparatus" may refer to any individual device, collection of devices, part of a device, or collections of parts of devices. The drawings are not necessarily to scale.

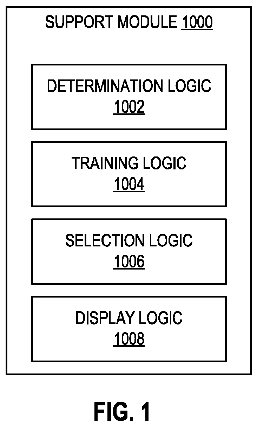

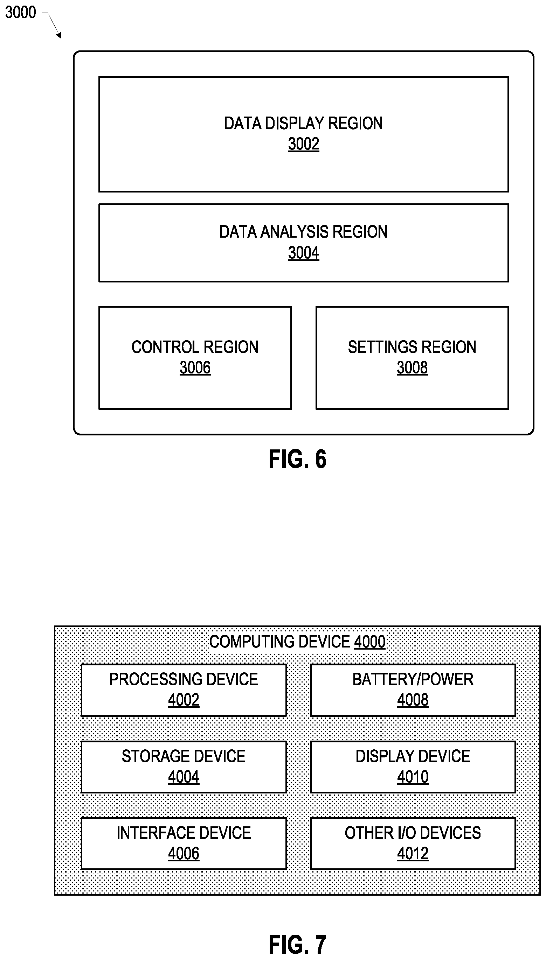

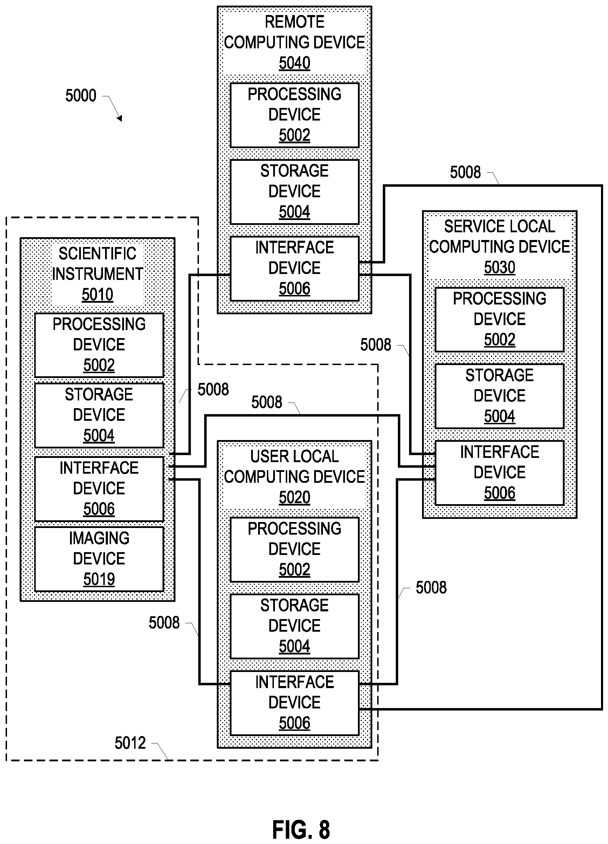

FIG. 1 is a block diagram of a scientific instrument support module 1000 for performing support operations, in accordance with various embodiments. The scientific instrument support module 1000 may be implemented by circuitry (e.g., including electrical and/or optical components), such as a programmed computing device. The logic of the scientific instrument support module 1000 may be included in a single computing device or may be distributed across multiple computing devices that are in communication with each other as appropriate. Examples of computing devices that may, singly or in combination, implement the scientific instrument support module 1000 are discussed herein with reference to the computing device 4000 of FIG. 7, and examples of systems of interconnected computing devices, in which the scientific instrument support module 1000 may be implemented across one or more of the computing devices, is discussed herein with reference to the scientific instrument support system 5000 of FIG. 8.

The scientific instrument support module 1000 may include determination logic 1002, training logic 1004, model selection logic 1006, and display logic 1008. As used herein, the term "logic" may include an apparatus that is to perform a set of operations associated with the logic. For example, any of the logic elements included in the support module 1000 may be implemented by one or more computing devices programmed with instructions to cause one or more processing devices of the computing devices to perform the associated set of operations. In a particular embodiment, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform one or more functions associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

The determination logic 1002 may be configured to determine a classification for each of a plurality of capsids in a biological specimen. The classification of each capsid may be determined based on received Cryo-EM data regarding the biological specimen, such as, for example, cryogenic transmission electron microscopy (Cryo-TEM) data obtained using a transmission electron microscope (TEM). In some embodiments, the determination logic 1002 employs a trained AI model to determine a classification of each capsid. As noted above, the determination logic 1002 may include an AI model that is trained with annotated data or assisted annotated data (supervised machine learning) provided by an individual or institution and reflects that individual's or institution's preferences.

In some embodiments, the AI model receives, as an input, Cryo-EM data regarding a biological specimen having a plurality of capsids, and outputs a classification (or a probability to belong to a class) for each of a plurality of capsids in a biological specimen.

The architecture of the AI model may take any of a number of forms, such as conventional machine learning (e.g., Gaussian mixtures, support vector machine, random forest, and the like.) or deep learning (e.g., deep convolutional neural networks) Training of the AI model included in the determination logic 1002 is discussed further below with reference to the training logic 1004.

The training logic 1004 may be configured to initially train the AI model used by the determination logic 1002 (e.g., trains a model with data augmentation and can thus be generalized to outputs the percentage of empty/partial/full capsids on various microscopes and various acquisition conditions), and to retrain the AI model upon the receipt of additional Cryo-EM data. Any suitable training technique for AI models may be implemented by the training logic 1004, such as data augmentation or a gradient descent process using suitable loss functions. In some embodiments, the AI model is a conventional machine learning model trained, by the training logic 1004, using deep learning where features are automatically extracted using deep convolutional layers (e.g., when a ResNet model is used). In some such examples, these features are computed prior to training the AI model. In some embodiments, capsids may be identified in a 2D image using an object detection algorithm, such as the YOLO algorithm. In some embodiments, in a supervised annotation step, features are primarily based on direct greylevels on a 2D image patch of the capsids (2D), using a radial averaged density profiles (1D), or the greylevel statistics of the capsids (e.g., mean, standard deviation, skew, kurtosis, and the like). In some embodiments, features are calculated inside the capsids (e.g., inside the shell), outside the capsids (e.g., background), or as a custom combination. In some embodiments, the emptiness of a capsid is defined according to:

$$E = (Min - Mshell)/(Min - Mout)$$

where Min is the mean intensity inside the shell, Mshell is the mean intensity of the shell, Mout is the mean intensity outside of the shell. Generally, E will tend to 1 for an empty capsid and tend to 0 for a full capsid. In some embodiments, a dimensionality reduction technique, such as principal component analysis, is employed to improve visualization capabilities via 2D or 3D point clouds.

In some embodiments, to retrain the AI model, the training logic 1004 may employ dropout or other regularization methods and may reserve some of the training data for use in validation and in assessing when to stop the retraining process. The training logic 1004 may perform such retraining on a regular chronological schedule (e.g., every week), after a certain number of confirmed image data sets or diagnostic and trending data sets are accumulated (e.g., 20), in accordance with any other suitable schedule, or at the command of a user (e.g., received via a GUI, such as the GUI 3000 of FIG. 6).

The model selection logic 1006 may be configured to provide multiple AI models that may be selectively utilized by the determination logic 1002 to determine a classification for each of a plurality of capsids in a biological specimen. For example, the AI models may be trained for various acquisition conditions. The display logic 1008 may provide to a user, through a GUI (such as the GUI 3000 of FIG. 6), an option to select the AI model that they wish to use for a particular acquisition condition from a set of stored AI models (e.g., identified with different names) made available by the model selection logic 1006, and the selected AI model may be used by the determination logic 1002 as part of determining a classification for each of a plurality of capsids in a biological specimen. The model selection logic 1006 may also provide to a user, through a GUI (such as the GUI 3000 of FIG. 6), an option to create a new AI model; the model selection logic 1006 may prompt a user to enter a name or other identifier for the new AI model, and to specify data that can be used to train the AI model. In some embodiments, the model selection logic 1006 may require a threshold amount of training data before training of a new AI model may proceed, and once trained, the new AI model may be available for selection via the model selection logic 1006.

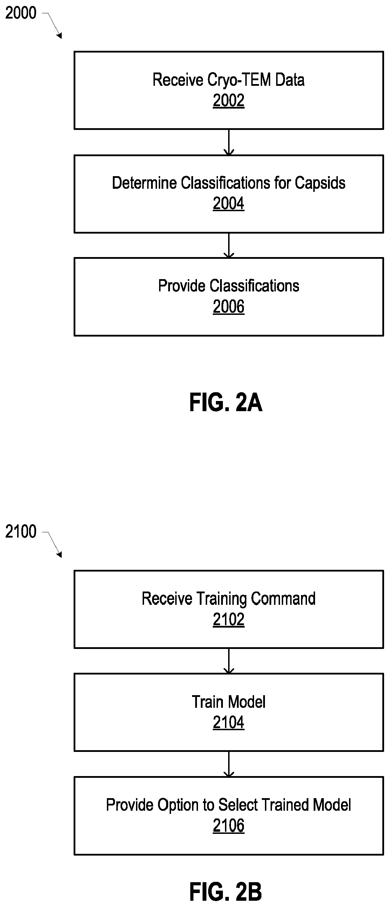

FIGS. 2A and 2B each depict a flow diagram of a method 2000 and 2100, respectively, of performing support operations, in accordance with various embodiments. Although the operations of the methods 2000 and 2100 may be illustrated with reference to particular embodiments disclosed herein (e.g., the scientific instrument support modules 1000 discussed herein with reference to FIG. 1, the GUI 3000 discussed herein with reference to FIG. 6, the computing devices 4000 discussed herein with reference to FIG. 7, and/or the scientific instrument support system 5000 discussed herein with reference to FIG. 8), the methods 2000 and 2100 may be used in any suitable setting to perform any suitable support operations. Operations are illustrated once each and in a particular order in FIGS. 2A and 2B, but the operations may be reordered and/or repeated as desired and appropriate (e.g., different operations performed may be performed in parallel, as suitable).

For method 2000, at 2002, first operations may be performed. For example, the support module 1000 may perform the operations of 2002 (e.g., via receiving logic not shown in FIG. 1). The first operations may include receiving, from a device, Cryo-EM data regarding a biological specimen having a plurality of capsids.

At 2004, second operations may be performed. For example, the determination logic 1002 of a support module 1000 may perform the operations of 2004. The second operations may include determining a classification for each of the capsids by processing the Cryo-EM data through an AI model trained with annotated data.

At 2006, third operations may be performed. For example, the display logic 1008 of a support module 1000 may perform the operations of 2006. The third operations may include displaying the classifications of the capsids in the biological specimen. In some embodiments, the classifications of the capsids generated at 2006 may be further used by the support module 1000 to make a quality determination on the biological specimen (e.g., when the percentage of capsids classified as "full" meets or exceeds a threshold or other set of conditions, the associated biological specimen may be identified as having appropriate quality for a particular purpose) and may cause the biological specimen to be routed or otherwise appropriately dispositioned (e.g., "high" quality specimens may be routed or labeled for use in certain applications, "medium" quality specimens may be routed or labeled for use in other applications, "low" quality specimens may be discarded, etc.).

For method 2100, at 2102, first operations may be performed. For example, the receiving logic (described above) of a support module 1000 may perform the operations of 2102. The first operations may include receiving a command to train an AI model. In some embodiments, the command includes an identification of a plurality of micrographs generated from an acquisition.

At 2104, second operations may be performed. For example, the training logic 1004 of a support module 1000 may perform the operations of 2104. The second operations may include annotating the micrographs using supervised learning models.

At 2106, third operations may be performed. For example, the training logic 1004 of a support module 1000 may perform the operations of 2106. The third operations may include training the AI model with the annotated micrographs to classify capsids.

At 2108, fourth operations may be performed. For example, the selection logic 1006 of a support module 1000 may perform the operations of 2108. The fourth operations may include providing, after training, an option to select the AI model for application to Cryo-EM data regarding a biological specimen.

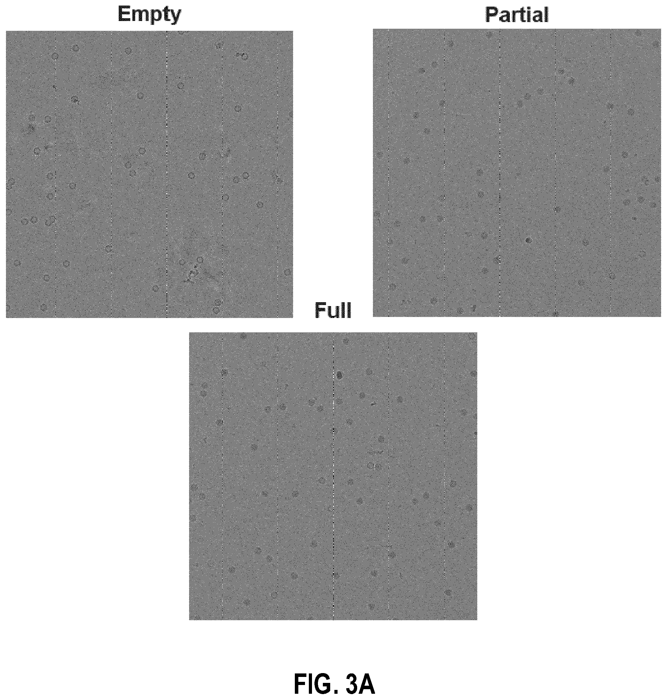

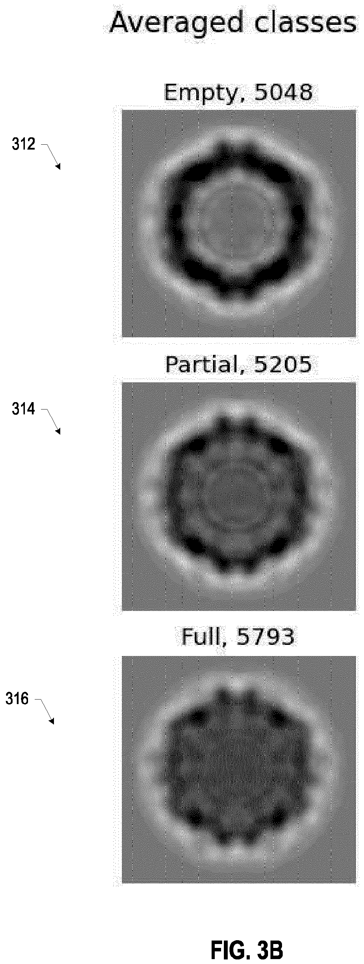

FIG. 3A depicts micrographs containing empty, partial, and full capsids. FIG. 3B depicts an example of an empty capsid 312, a partially filed capsid 314, and a full capsid 316. In some embodiments, the described system employs supervised machine learning on reference stocks of capsids (empty/partial/full, E/P/F) to semi-automatically annotate micrographs to further train a generic model capable of distinguish empty, partial, and full capsids in a broad range of acquisition conditions, benefiting from data augmentation. Supervised machine learning workflows may be computationally intensive because they may be required to calculate many features. These workflows are also difficult to generalize and require to be supervised for every new dataset.

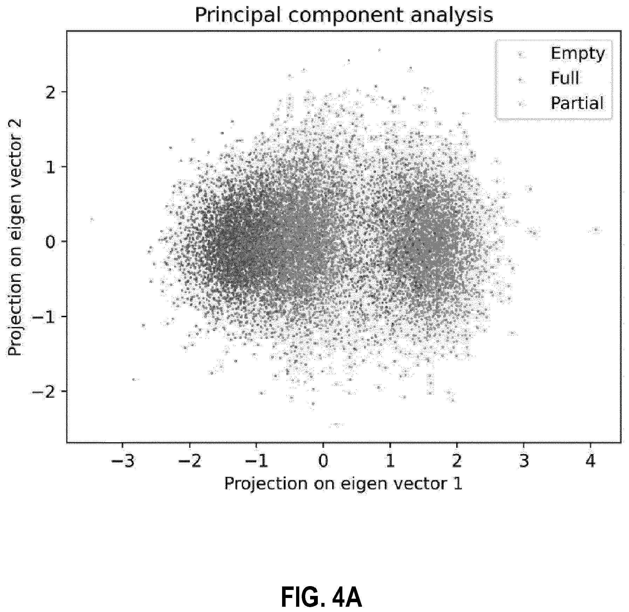

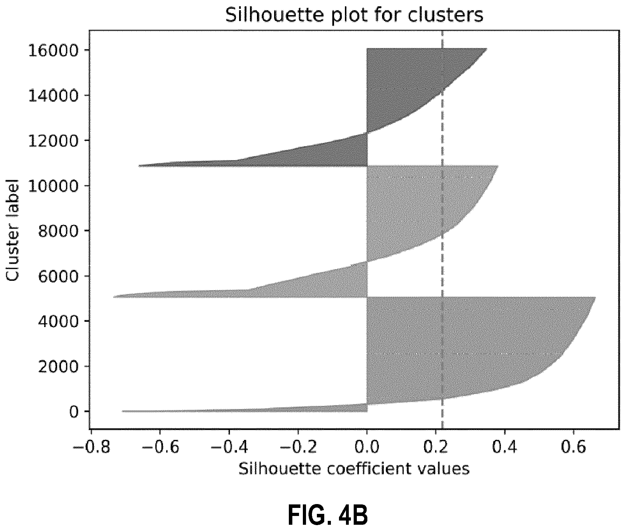

A PCA clustering illustration is depicted in FIG. 4A (with "full" points largely on the left, "empty" points largely on the right, and "partial" points largely in the middle) while corresponding silhouette scores are depicted in FIG. 4B (with "full" at the top, "empty" at the bottom, and "partial" in the middle) for capsid data classified using the AI techniques disclosed herein. Generally, preparation of AAV samples needs to be carefully characterized to ensure product quality, efficacy, and safety. Features of interest include concentration, aggregation, capsid content (e.g., full/partial/empty), purity (e.g., residual DNA, proteins, and the like) and integrity (e.g., broken, doublets, triplets). Standard methods include analytical ultracentrifugation (AUC), ELISA, SEC-MALS, and mass spectroscopy.

When analyzing clusters, >20% of outliers in the partial and full stocks were detected (later confirmed with AUC (ultracentrifugation) values). These results show that Cryo-EM combined with computer vision is a powerful method for nanoparticles characterization, Cryo-EM imaging may provide a direct access to capsids and multiplexed analysis, Cryo-EM imaging coupled with machine learning enables differentiation of E/P/F capsids, and Cryo-EM has better sensitivity than AUC.

The benefits of using Cryo-EM in the characterization of AAV samples (e.g., using Cryo-TEM) include providing a cryogenic condition, an unperturbed sample in native condition, direct visualization of capsids and cargos, direct accurate measurement, access to multiplex critical attributes, and a small sample requirement (e.g., $3\mu L$). Also, Cryo-EM can support process development by confirming purity and status of the viral capsids. Additionally, Cryo-EM may also be used with other common types of analyses (e.g., single particle analysis (SPA), tomography, microcrystal electron diffraction (MicroED), so that using Cryo-EM data for characterization of AAV samples may enable greater overall scientific throughput.

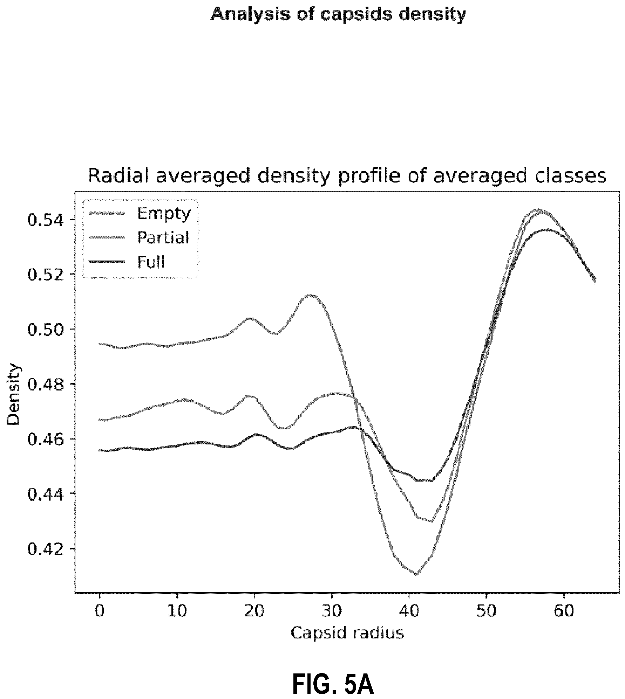

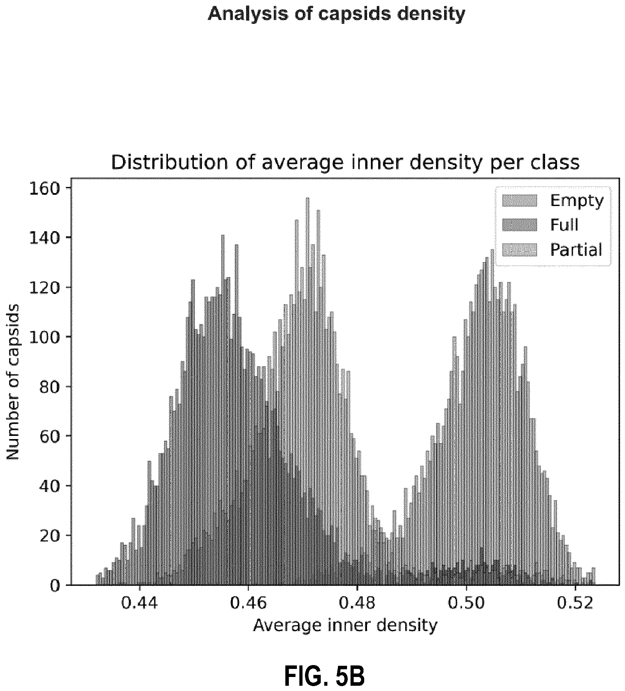

FIGS. 5A and 5B show the results of statistical analysis of density using the described system. FIG. 5A depicts a chart showing a radial averaged density profile of averaged classes. FIG. 5B depicts a chart showing a distribution of average inner density per class (with the "full" distribution largely at the left, the "empty" distribution largely at the right, and the "partial" distribution largely in the middle). In the depicted results, for each stock, corresponding average capsids and density profiles were computed. Three levels of inner density with sufficient contrast to distinguish empty, partial, full are shown as well as histograms of inner density. The results show three distinct distributions, with contrasted peaks, showing that Cryo-EM provides enough contrast to distinguish empty, partial, and filled capsids.

The scientific instrument support methods disclosed herein may include interactions with a human user (e.g., via the user local computing device 5020 discussed herein with reference to FIG. 8). These interactions may include providing information to the user (e.g., information regarding the operation of a scientific instrument such as the scientific instrument 5010 of FIG. 8, information regarding a sample being analyzed or other test or measurement performed by a scientific instrument, information retrieved from a local or remote database, or other information) or providing an option for a user to input commands (e.g., to control the operation of a scientific instrument such as the scientific instrument 5010 of FIG. 8, or to control the analysis of data generated by a scientific instrument), queries (e.g., to a local or remote database), or other information. In some embodiments, these interactions may be performed through a graphical user interface (GUI) that includes a visual display on a display device (e.g., the display device 4010 discussed herein with reference to FIG. 7) that provides outputs to the user and/or prompts the user to provide inputs (e.g., via one or more input devices, such as a keyboard, mouse, trackpad, or touchscreen, included in the other I/O devices 4012 discussed herein with reference to FIG. 7). The scientific instrument support systems disclosed herein may include any suitable GUIs for interaction with a user.

FIG. 6 depicts an example GUI 3000 that may be used in the performance of some or all of the support methods disclosed herein, in accordance with various embodiments. As noted above, the GUI 3000 may be provided on a display device (e.g., the display device 4010 discussed herein with reference to FIG. 7) of a computing device (e.g., the computing device 4000 discussed herein with reference to FIG. 7) of a scientific instrument support system (e.g., the scientific instrument support system 5000 discussed herein with reference to FIG. 8), and a user may interact with the GUI 3000 using any suitable input device (e.g., any of the input devices included in the other I/O devices 4012 discussed herein with reference to FIG. 7) and input technique (e.g., movement of a cursor, motion capture, facial recognition, gesture detection, voice recognition, actuation of buttons, etc.).

The GUI 3000 may include a data display region 3002, a data analysis region 3004, a scientific instrument control region 3006, and a settings region 3008. The particular number and arrangement of regions depicted in FIG. 6 is simply illustrative, and any number and arrangement of regions, including any desired features, may be included in a GUI 3000. The data display region 3002 may display data generated by a scientific instrument (e.g., the scientific instrument 5010 discussed herein with reference to FIG. 8).

The data analysis region 3004 may display the results of data analysis (e.g., the results of analyzing the data illustrated in the data display region 3002 and/or other data). For example, the data analysis region 3004 may display state information that includes the classifications of the capsids in the biological specimen determined by system processing Cryo-EM data regarding the biological specimen through a trained model. In some embodiments, the data display region 3002 and the data analysis region 3004 may be combined in the GUI 3000 (e.g., to include data output from a scientific instrument, and some analysis of the data, in a common graph or region).

The scientific instrument control region 3006 may include options that allow the user to control a scientific instrument (e.g., the scientific instrument 5010 discussed herein with reference to FIG. 8). The settings region 3008 may include options that allow the user to control the features and functions of the GUI 3000 (and/or other GUIs) and/or perform common computing operations with respect to the data display region 3002 and data analysis region 3004 (e.g., saving data on a storage device, such as the storage device 4004 discussed herein with reference to FIG. 7, sending data to another user, labeling data, etc.).

As noted above, the scientific instrument support module 1000 may be implemented by one or more computing devices. FIG. 7 is a block diagram of a computing device 4000 that may perform some or all of the scientific instrument support methods disclosed herein, in accordance with various embodiments. In some embodiments, the scientific instrument support module 1000 may be implemented by a single computing device 4000 or by multiple computing devices 4000. Further, as discussed below, a computing device 4000 (or multiple computing devices 4000) that implements the scientific instrument support module 1000 may be part of one or more of the scientific instrument 5010, the user local computing device 5020, the service local computing device 5030, or the remote computing device 5040 of FIG. 8.

The computing device 4000 of FIG. 7 is illustrated as having a number of components, but any one or more of these components may be omitted or duplicated, as suitable for the application and setting. In some embodiments, some or all of the components included in the computing device 4000 may be attached to one or more motherboards and enclosed in a housing (e.g., including plastic, metal, and/or other materials). In some embodiments, some these components may be fabricated onto a single system-on-a-chip (SoC) (e.g., an SoC may include one or more processing devices 4002 and one or more storage devices 4004). Additionally, in various embodiments, the computing device 4000 may not include one or more of the components illustrated in FIG. 7, but may include interface circuitry (not shown) for coupling to the one or more components using any suitable interface (e.g., a Universal Serial Bus (USB) interface, a High-Definition Multimedia interface (HDMI) interface, a Controller Area Network (CAN) interface, a Serial Peripheral interface (SPI) interface, an Ethernet interface, a wireless interface, or any other appropriate interface) . For example, the computing device 4000 may not include a display device 4010, but may include display device interface circuitry (e.g., a connector and driver circuitry) to which a display device 4010 may be coupled.

The computing device 4000 may include a processing device 4002 (e.g., one or more processing devices). As used herein, the term "processing device" may refer to any device or portion of a device that processes electronic data from registers and/or memory to transform that electronic data into other electronic data that may be stored in registers and/or memory. The processing device 4002 may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 4000 may include a storage device 4004 (e.g., one or more storage devices). The storage device 4004 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 4004 may include memory that shares a die with a processing device 4002. In such an embodiment, the memory may be used as cache memory and may include embedded dynamic random access memory (eDRAM) or spin transfer torque magnetic random access memory (STT-MRAM), for example. In some embodiments, the storage device 4004 may include non-transitory computer readable media having instructions thereon that, when executed by one or more processing devices (e.g., the processing device 4002), cause the computing device 4000 to perform any appropriate ones of or portions of the methods disclosed herein.

The computing device 4000 may include an interface device 4006 (e.g., one or more interface devices 4006). The interface device 4006 may include one or more communication chips, connectors, and/or other hardware and software to govern communications between the computing device 4000 and other computing devices. For example, the interface device 4006 may include circuitry for managing wireless communications for the transfer of data to and from the computing device 4000. The term "wireless" and its derivatives may be used to describe circuits, devices, systems, methods, techniques, communications channels, and the like., that may communicate data through the use of modulated electromagnetic radiation through a nonsolid medium. The term does not imply that the associated devices do not contain any wires, although in some embodiments they might not. Circuitry included in the interface device 4006 for managing wireless communications may implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra-mobile broadband (UMB) project (also referred to as "3GPP2"), etc.). In some embodiments, circuitry included in the interface device 4006 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 4006 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 4006 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 4006 may include one or more antennas (e.g., one or more antenna arrays) to receipt and/or transmission of wireless communications.

In some embodiments, the interface device 4006 may include circuitry for managing wired communications, such as electrical, optical, or any other suitable communication protocols. For example, the interface device 4006 may include circuitry to support communications in accordance with Ethernet technologies. In some embodiments, the interface device 4006 may support both wireless and wired communication, and/or may support multiple wired communication protocols and/or multiple wireless communication protocols. For example, a first set of circuitry of the interface device 4006 may be dedicated to shorter-range wireless communications such as Wi-Fi or Bluetooth, and a second set of circuitry of the interface device 4006 may be dedicated to longer-range wireless communications such as global positioning system (GPS), EDGE, GPRS, CDMA, WiMAX, LTE, EV-DO, or others. In some embodiments, a first set of circuitry of the interface device 4006 may be dedicated to wireless communications, and a second set of circuitry of the interface device 4006 may be dedicated to wired communications.

The computing device 4000 may include battery/power circuitry 4008. The battery/power circuitry 4008 may include one or more energy storage devices (e.g., batteries or capacitors) and/or circuitry for coupling components of the computing device 4000 to an energy source separate from the computing device 4000 (e.g., AC line power).

The computing device 4000 may include a display device 4010 (e.g., multiple display devices). The display device 4010 may include any visual indicators, such as a heads-up display, a computer monitor, a projector, a touchscreen display, a liquid crystal display (LCD), a light-emitting diode display, or a flat panel display.

The computing device 4000 may include other input/output (I/O) devices 4012. The other I/O devices 4012 may include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 4000, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 4000 may have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultramobile personal computer, etc.), a desktop computing device, or a server computing device or other networked computing component.

One or more computing devices implementing any of the scientific instrument support modules or methods disclosed herein may be part of a scientific instrument support system. FIG. 8 is a block diagram of an example scientific instrument support system 5000 in which some or all of the scientific instrument support methods disclosed herein may be performed, in accordance with various embodiments. The scientific instrument support modules and methods disclosed herein (e.g., the scientific instrument support module 1000 of FIG. 1 and the methods 2000 and 2100 of FIGS. 2A and 2B respectively) may be implemented by one or more of the scientific instrument 5010, the user local computing device 5020, the service local computing device 5030, or the remote computing device 5040 of the scientific instrument support system 5000.

Any of the scientific instrument 5010, the user local computing device 5020, the service local computing device 5030, or the remote computing device 5040 may include any of the embodiments of the computing device 4000 discussed herein with reference to FIG. 7, and any of the scientific instrument 5010, the user local computing device 5020, the service local computing device 5030, or the remote computing device 5040 may take the form of any appropriate ones of the embodiments of the computing device 4000 discussed herein with reference to FIG. 7.

The scientific instrument 5010, the user local computing device 5020, the service local computing device 5030, or the remote computing device 5040 may each include a processing device 5002, a storage device 5004, and an interface device 5006. The processing device 5002 may take any suitable form, including the form of any of the processing devices 4002 discussed herein with reference to FIG. 4, and the processing devices 5002 included in different ones of the scientific instrument 5010, the user local computing device 5020, the service local computing device 5030, or the remote computing device 5040 may take the same form or different forms. The storage device 5004 may take any suitable form, including the form of any of the storage devices 5004 discussed herein with reference to FIG. 4, and the storage devices 5004 included in different ones of the scientific instrument 5010, the user local computing device 5020, the service local computing device 5030, or the remote computing device 5040 may take the same form or different forms. The interface device 5006 may take any suitable form, including the form of any of the interface devices 4006 discussed herein with reference to FIG. 4, and the interface devices 5006 included in different ones of the scientific instrument 5010, the user local computing device 5020, the service local computing device 5030, or the remote computing device 5040 may take the same form or different forms.

The scientific instrument 5010, the user local computing device 5020, the service local computing device 5030, and the remote computing device 5040 may be in communication with other elements of the scientific instrument support system 5000 via communication pathways 5008. The communication pathways 5008 may communicatively couple the interface devices 5006 of different ones of the elements of the scientific instrument support system 5000, as shown, and may be wired or wireless communication pathways (e.g., in accordance with any of the communication techniques discussed herein with reference to the interface devices 4006 of the computing device 4000 of FIG. 7). The particular scientific instrument support system 5000 depicted in FIG. 8 includes communication pathways between each pair of the scientific instrument 5010, the user local computing device 5020, the service local computing device 5030, and the remote computing device 5040, but this "fully connected" implementation is simply illustrative, and in various embodiments, various ones of the communication pathways 5008 may be absent. For example, in some embodiments, a service local computing device 5030 may not have a direct communication pathway 5008 between its interface device 5006 and the interface device 5006 of the scientific instrument 5010, but may instead communicate with the scientific instrument 5010 via the communication pathway 5008 between the service local computing device 5030 and the user local computing device 5020 and the communication pathway 5008 between the user local computing device 5020 and the scientific instrument 5010.

The scientific instrument 5010 may include any appropriate scientific instrument. In some embodiments, the scientific instrument 5010 includes a cryogenic (Cryo) electron microscope, such as, for example, a cryogenic electron transmission microscope (TEM). A TEM includes an electron source that produces a beam of electrons directed onto a specimen contained within a vacuum enclosure. A cooling device may be in thermal contact with a holder of the specimen to maintain the holder and the specimen thereupon) at cryogenic temperatures. The electron beam interacts with the specimen in such a manner as to cause various types of "stimulated" radiation to emanate from the specimen, including (for example) secondary electrons, backscattered electrons, X-rays, and optical radiation (cathodoluminescence). If desired, one or more of these radiation types can be detected with the aid of analysis device, which might be a combined scintillator/photomultiplier or EDX (Energy-Dispersive X-Ray Spectroscopy) module, for instance; in such a case, an image could be constructed. However, alternatively or supplementally, one can study electrons that traverse (pass through) the specimen, exit/emanate from the specimen, and continue to propagate (substantially, though generally with some deflection/scattering). Such a transmitted electron flux enters an imaging system (projection lens), which can generally comprise a variety of electrostatic / magnetic lenses, deflectors, correctors (such as, for example, stigmators), and the like. In a non-scanning TEM mode, this imaging system can focus the transmitted electron flux onto a fluorescent screen, which, if desired, can be retracted or withdrawn. An image (or diffractogram) of (part of ) the specimen can be formed by the imaging system on a screen, which may be viewed through viewing port located, for example, in a suitable part of a wall of the vacuum enclosure.

As an alternative to viewing an image on screen, one can instead make use of the fact that the depth of focus of the electron flux leaving an imaging system is generally quite large (e.g., of the order of 1 meter). Consequently, various other types of analysis apparatus can be used downstream of screen, such as a TEM camera. At the TEM camera, the electron flux can form a static image (or diffractogram) that can be processed by a controller or processor (e.g., computing device 4000) (local to the scientific instrument or remote from scientific instrument) and displayed on a display device, such as a flat panel display, for example. When not required, the camera may be configured to be retracted or withdrawn. As an alternative to imaging using a camera, one can also invoke spectroscopic apparatus, which could be an electron energy loss spectroscopy (EELS) module, for example.

The user local computing device 5020 may be a computing device (e.g., in accordance with any of the embodiments of the computing device 4000 discussed herein) that is local to a user of the scientific instrument 5010. In some embodiments, the user local computing device 5020 may also be local to the scientific instrument 5010, but this need not be the case; for example, a user local computing device 5020 that is in a user's home or office may be remote from, but in communication with, the scientific instrument 5010 so that the user may use the user local computing device 5020 to control and/or access data from the scientific instrument 5010. In some embodiments, the user local computing device 5020 may be a laptop, smartphone, or tablet device. In some embodiments the user local computing device 5020 may be a portable computing device.

The service local computing device 5030 may be a computing device (e.g., in accordance with any of the embodiments of the computing device 4000 discussed herein) that is local to an entity that services the scientific instrument 5010. For example, the service local computing device 5030 may be local to a manufacturer of the scientific instrument 5010 or to a third-party service company. In some embodiments, the service local computing device 5030 may communicate with the scientific instrument 5010, the user local computing device 5020, and/or the remote computing device 5040 (e.g., via a direct communication pathway 5008 or via multiple "indirect" communication pathways 5008, as discussed above) to receive data regarding the operation of the scientific instrument 5010, the user local computing device 5020, and/or the remote computing device 5040 (e.g., the results of self-tests of the scientific instrument 5010, calibration coefficients used by the scientific instrument 5010, the measurements of sensors associated with the scientific instrument 5010, etc.). In some embodiments, the service local computing device 5030 may communicate with the scientific instrument 5010, the user local computing device 5020, and/or the remote computing device 5040 (e.g., via a direct communication pathway 5008 or via multiple "indirect" communication pathways 5008, as discussed above) to transmit data to the scientific instrument 5010, the user local computing device 5020, and/or the remote computing device 5040 (e.g., to update programmed instructions, such as firmware, in the scientific instrument 5010, to initiate the performance of test or calibration sequences in the scientific instrument 5010, to update programmed instructions, such as software, in the user local computing device 5020 or the remote computing device 5040, etc.). A user of the scientific instrument 5010 may utilize the scientific instrument 5010 or the user local computing device 5020 to communicate with the service local computing device 5030 to report a problem with the scientific instrument 5010 or the user local computing device 5020, to request a visit from a technician to improve the operation of the scientific instrument 5010, to order consumables or replacement parts associated with the scientific instrument 5010, or for other purposes.

The remote computing device 5040 may be a computing device (e.g., in accordance with any of the embodiments of the computing device 4000 discussed herein) that is remote from the scientific instrument 5010 and/or from the user local computing device 5020. In some embodiments, the remote computing device 5040 may be included in a datacenter or other large-scale server environment. In some embodiments, the remote computing device 5040 may include network-attached storage (e.g., as part of the storage device 5004). The remote computing device 5040 may store data generated by the scientific instrument 5010, perform analyses of the data generated by the scientific instrument 5010 (e.g., in accordance with programmed instructions), facilitate communication between the user local computing device 5020 and the scientific instrument 5010, and/or facilitate communication between the service local computing device 5030 and the scientific instrument 5010.

In some embodiments, one or more of the elements of the scientific instrument support system 5000 illustrated in FIG. 8 may not be present. Further, in some embodiments, multiple ones of various ones of the elements of the scientific instrument support system 5000 of FIG. 8 may be present. For example, a scientific instrument support system 5000 may include multiple user local computing devices 5020 (e.g., different user local computing devices 5020 associated with different users or in different locations). In another example, a scientific instrument support system 5000 may include multiple scientific instruments 5010, all in communication with service local computing device 5030 and/or a remote computing device 5040; in such an embodiment, the service local computing device 5030 may monitor these multiple scientific instruments 5010, and the service local computing device 5030 may cause updates or other information may be "broadcast" to multiple scientific instruments 5010 at the same time. Different ones of the scientific instruments 5010 in a scientific instrument support system 5000 may be located close to one another (e.g., in the same room) or farther from one another (e.g., on different floors of a building, in different buildings, in different cities, and the like.). In some embodiments, a scientific instrument 5010 may be connected to an Internet-of-Things (IoT) stack that allows for command and control of the scientific instrument 5010 through a web-based application, a virtual or augmented reality application, a mobile application, and/or a desktop application. Any of these applications may be accessed by a user operating the user local computing device 5020 in communication with the scientific instrument 5010 by the intervening remote computing device 5040. In some embodiments, a scientific instrument 5010 may be sold by the manufacturer along with one or more associated user local computing devices 5020 as part of a local scientific instrument computing unit 5012.

In some such embodiments, the remote computing device 5040 and/or the user local computing device 5020 may combine data from different types of scientific instruments 5010 included in a scientific instrument support system 5000.

The following paragraphs provide various examples of the embodiments disclosed herein.

Example 1 is a scientific instrument support apparatus including first logic to first logic to receive, from a cryo electron microscope, Cryo-EM data regarding a biological specimen having a plurality of capsids; second logic to determine a classification for each of the capsids by processing the Cryo-EM data through an AI model trained with annotated data; and third logic to display the classifications of the capsids in the biological specimen.

Example 2 includes the subject matter of Example 1, and further specifies that the biological specimen comprises an AAV.

Example 3 includes the subject matter of any of Examples 1 and 2, and further specifies that the biological specimen is vitrified by rapid freezing to integrate molecules into an amorphous ice.

Example 4 includes the subject matter of any of Examples 1-3, and further specifies that the capsids are classified as empty, partially filled, or filled.

Example 5 includes the subject matter of any of Examples 1-4, and further specifies that the second logic is further configured to determine the purity of the biological sample based on the determined capsid classifications.

Example 6 includes the subject matter of any of Examples 1-5, and further specifies that the annotated data comprises annotated micrographs, where the micrographs are annotated using supervised learning models.

Example 7 includes the subject matter of any of Examples 1-6, and further specifies that trained AI model is capable of distinguish empty, partial, and full capsids in a range of acquisition conditions.

Example 8 is a scientific instrument support apparatus including first logic to receive a command to train an AI model, where the command includes an identification of a plurality of micrographs generated from an acquisition; second logic to annotate the micrographs using supervised learning models; third logic to train the AI model with the annotated micrographs to classify capsids; and fourth logic to provide, after training, an option to select the AI model for application to Cryo-EM data regarding a biological specimen.

Example 9 includes the subject matter of Example 8, and further specifies that the AI model is capable of distinguish empty, partial, and full capsids in a range of acquisition conditions.

Example 10 includes the subject matter of any of Examples 8 or 9, and further specifies that the second logic is further configured to normalize, mask, align, and extract features from the capsids identified in the micrographs.

Example 11 includes the subject matter of any of Examples 8-10, and further specifies that the capsids are classified as empty, partially filled, or filled.

Example 12 includes the subject matter of any of Examples 8-11, and further specifies that the acquisition comprises an electronic image (micrograph) of a cross-section of a biological sample.

Example 13 includes the subject matter of any of Examples 8-14, and further specifies that the biological sample comprises nanoparticles or viral vectors.

Example 14 includes the subject matter of any of Examples 8-13, and further specifies that the viral vectors comprise Adeno Associated Viruses or Lipid Nano Particles.

Example 15 is a scientific instrument support apparatus, including: first logic to receive, from a cryogenic (Cryo) electron microscope, Cryo-electron microscopy (Cryo-EM) data regarding a biological specimen having a plurality of capsids; second logic to determine a classification for each of the capsids by processing the Cryo-EM data through an artificial intelligence (AI) model trained with annotated data; and third logic to output the classifications of the capsids in the biological specimen.

Example 16 includes the subject matter of Example 15, and further specifies that the biological specimen comprises an adeno associated virus (AAV).

Example 17 includes the subject matter of any of Examples 15-16, and further specifies that the biological specimen is vitrified by rapid freezing to integrate molecules into an amorphous ice.

Example 18 includes the subject matter of any of Examples 15-17, and further specifies that the capsid classifications are selected from a set of possible classifications that include empty and filled.

Example 19 includes the subject matter of Example 18, and further specifies that the set of possible classifications includes partially filled.

Example 20 includes the subject matter of any of Examples 15-19, and further specifies that the second logic is further configured to determine the purity of the biological sample based on the determined capsid classifications.

Example 21 includes the subject matter of any of Examples 15-20, and further specifies that the annotated data comprises annotated micrographs, where the micrographs are annotated using supervised learning models.

Example 22 is a scientific instrument support apparatus, including: first logic to receive a command to train an artificial intelligence (AI) model, wherein the command includes an identification of a plurality of micrographs generated by an electron microscope; second logic to annotate the micrographs using a supervised learning method; third logic to train the AI model with the annotated micrographs to classify capsids; and fourth logic to provide, after training, an option to select the AI model for application to cryo-electron microscopy (Cryo-EM) data regarding a biological specimen.

Example 23 includes the subject matter of Example 22, and further specifies that the second logic is further configured to normalize, mask, align, and extract features from the capsids identified in the micrographs.

Example 24 includes the subject matter of any of Examples 22-23, and further specifies that the possible capsid classifications include empty and filled.

Example 25 includes the subject matter of Example 24, and further specifies that the possible capsid classifications include partially filled.

Example 26 includes the subject matter of any of Examples 22-25, and further specifies that the acquisition comprises an electronic image (micrograph) of a cross-section of a biological sample.

Example 27 includes the subject matter of Example 26, and further specifies that the biological sample comprises nanoparticles or viral vectors.

Example 28 includes the subject matter of Example 27, and further specifies that the viral vectors comprise Adeno Associated Viruses or Lipid Nano Particles.

Example 29 is a capsid quality control method, including: receiving, from a microscope, microscopy data regarding a biological specimen having a plurality of capsids; determining a classification for each of the capsids by processing the microscopy data through an artificial intelligence (AI) model trained with annotated data; and outputting a quality assessment of the biological specimen based on the determined capsid classifications.

Example 30 includes the subject matter of Example 29, and further specifies that the biological specimen comprises an adeno associated virus (AAV).

Example 31 includes the subject matter of any of Examples 29-30, and further specifies that the microscopy data is cryo-electron microscopy (Cryo-EM) data.

Example 32 includes the subject matter of any of Examples 29-31, further including vitrifying the biological specimen.

Example 33 includes the subject matter of Example 32, further including causing the microscope to acquire images of the vitrified biological specimen, wherein the images are included in the microscopy data.

Example 34 includes the subject matter of Example 33, and further specifies that the capsid classifications are selected from a set of possible classifications that include empty and filled.

**Claims**

1. A scientific instrument support apparatus, comprising:

   first logic to receive, from a cryogenic (Cryo) electron microscope, Cryo-electron microscopy (Cryo-EM) data regarding a biological specimen having a plurality of capsids;

second logic to determine a classification for each of the capsids by processing the Cryo-EM data through an artificial intelligence (AI) model trained with annotated data; and

third logic to output the classifications of the capsids in the biological specimen.

2. The scientific instrument support apparatus of claim 1, wherein the biological specimen comprises an adeno associated virus (AAV).

3. The scientific instrument support apparatus of claim 1, wherein the biological specimen is vitrified by rapid freezing to integrate molecules into an amorphous ice.

4. The scientific instrument support apparatus of claim 1, wherein the capsid classifications are selected from a set of possible classifications that include empty and filled; and optionally wherein the set of possible classifications includes partially filled.

5. The scientific instrument support apparatus of claim 1, wherein the second logic is further configured to determine the purity of the biological sample based on the determined capsid classifications.

6. The scientific instrument support apparatus of claim 1, wherein the annotated data comprises annotated micrographs, where the micrographs are annotated using supervised learning models.

7. A scientific instrument support apparatus, comprising:

first logic to receive a command to train an artificial intelligence (AI) model, wherein the command includes an identification of a plurality of micrographs generated by an electron microscope;

second logic to annotate the micrographs using a supervised learning method;

third logic to train the AI model with the annotated micrographs to classify capsids; and

fourth logic to provide, after training, an option to select the AI model for application to cryo-electron microscopy (Cryo-EM) data regarding a biological specimen.

8. The scientific instrument support apparatus of claim 7, wherein the second logic is further configured to normalize, mask, align, and extract features from the capsids identified in the micrographs.

9. The scientific instrument support apparatus of claim 7, wherein the possible capsid classifications include empty and filled; and optionally wherein the possible capsid classifications include partially filled.

10. The scientific instrument support apparatus of claim 7, wherein the acquisition comprises an electronic image (micrograph) of a cross-section of a biological sample.

11. The scientific instrument support apparatus of claim 10, wherein the biological sample comprises nanoparticles or viral vectors; and optionally wherein the viral vectors comprise Adeno Associated Viruses or Lipid Nano Particles.

12. A capsid quality control method, comprising:

receiving, from a microscope, microscopy data regarding a biological specimen having a plurality of capsids;

determining a classification for each of the capsids by processing the microscopy data through an artificial intelligence (AI) model trained with annotated data; and

outputting a quality assessment of the biological specimen based on the determined capsid classifications.

13. The capsid quality control method of claim 12, wherein the biological specimen comprises an adeno associated virus (AAV).

14. The capsid quality control method of claim 12, wherein the microscopy data is cryo-electron microscopy (Cryo-EM) data.

15. The capsid quality control method of claim 12, further comprising:
vitrifying the biological specimen; and optionally further comprising:
causing the microscope to acquire images of the vitrified biological specimen, wherein the images are included in the microscopy data; and optionally wherein the capsid classifications are selected from a set of possible classifications that include empty and filled.

SUPPORT MODULE 1000

DETERMINATION LOGIC
1002

TRAINING LOGIC
1004

SELECTION LOGIC
1006

DISPLAY LOGIC
1008

**FIG. 1**

2000

```
┌─────────────────────────────┐
│   Receive Cryo-TEM Data     │
│           2002              │
└─────────────────────────────┘
              │
              ▼
┌─────────────────────────────┐
│ Determine Classifications   │
│       for Capsids           │
│           2004              │
└─────────────────────────────┘
              │
              ▼
┌─────────────────────────────┐
│   Provide Classifications   │
│           2006              │
└─────────────────────────────┘
```

**FIG. 2A**

2100

```
┌─────────────────────────────┐
│  Receive Training Command   │
│           2102              │
└─────────────────────────────┘
              │
              ▼
┌─────────────────────────────┐
│        Train Model          │
│           2104              │
└─────────────────────────────┘
              │
              ▼
┌─────────────────────────────┐
│ Provide Option to Select    │
│      Trained Model          │
│           2106              │
└─────────────────────────────┘
```

**FIG. 2B**

Empty

Partial

Full

FIG. 3A

# Averaged classes

### Empty, 5048

312

### Partial, 5205

314

### Full, 5793

316

**FIG. 3B**

FIG. 4A

Silhouette plot for clusters

FIG. 4B

**Analysis of capsids density**

FIG. 5A

**Analysis of capsids density**

Distribution of average inner density per class

FIG. 5B

3000

DATA DISPLAY REGION
3002

DATA ANALYSIS REGION
3004

CONTROL REGION
3006

SETTINGS REGION
3008

**FIG. 6**

COMPUTING DEVICE 4000

PROCESSING DEVICE
4002

BATTERY/POWER
4008

STORAGE DEVICE
4004

DISPLAY DEVICE
4010

INTERFACE DEVICE
4006

OTHER I/O DEVICES
4012

**FIG. 7**

**FIG. 8**

Europäisches
Patentamt

European
Patent Office

Office européen
des brevets

# EUROPEAN SEARCH REPORT

Application Number

EP 24 16 0837

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 112 712 031 A (SHUIMU BIOSCIENCE LTD) 27 April 2021 (2021-04-27) * abstract * | 1-15 | INV. G06V10/82 G06V20/69 |
| A | CN 112 183 433 A (SHUIMU BIOSCIENCE LTD) 5 January 2021 (2021-01-05) * abstract * | 1-15 | |

TECHNICAL FIELDS
SEARCHED      (IPC)

G06V

The present search report has been drawn up for all claims

| Place of search | Date of completion of the search | Examiner |
|---|---|---|
| The Hague | 1 July 2024 | Sagrebin-Mitzel, M |

EPO FORM 1503 03.82 (P04C01)

## ANNEX TO THE EUROPEAN SEARCH REPORT
## ON EUROPEAN PATENT APPLICATION NO.

EP 24 16 0837

This annex lists the patent family members relating to the patent documents cited in  the above-mentioned European search report.
The members are as contained in the European Patent Office EDP file on
The European Patent Office is in no way liable for these particulars which are merely given for the purpose of information.

01-07-2024

| Patent document cited in search report | | Publication date | Patent family member(s) | Publication date |
|---|---|---|---|---|
| CN 112712031 | A | 27-04-2021 | NONE | |
| CN 112183433 | A | 05-01-2021 | NONE | |

EPO FORM P0459

For more details about this annex : see Official Journal of the European Patent Office, No. 12/82

**EP 4 425 446 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63487715
- US 63499645